# EUROPEAN PATENT SPECIFICATION

(11) **EP 3 362 054 B1**
(45) Date of publication and mention of the grant of the patent: **14.04.2021**
(21) Application number: 16798643.9
(22) Date of filing: 13.10.2016
(51) Int. Cl.: A61K 9/20, A61K 31/197

(54) **PREGABALIN COMPOSITIONS**
PREGABALINZUSAMMENSETZUNGEN
COMPOSITIONS DE PRÉGABALINE

(30) Priority: 14.10.2015 EP 15382504
(43) Date of publication of application: 22.08.2018
(73) Proprietor: Laboratorios Lesvi, S.L., 08970 Sant Joan Despí - Barcelona (ES)
(72) Inventor: RUIZ AMENOS, Anna, 08191 Sant Joan Despí, Barcelona (ES); UBEDA PEREZ, Carmen, 08970 Sant Joan Despí, Barcelona (ES); DIEZ MARTIN, Ignacio, 08970 Sant Joan Despí, Barcelona (ES)
(74) Representative: Isarpatent
(86) International application number: PCT/EP2016/074600
(87) International publication number: WO 2017/064192

(56) References cited:
- WO-A2-2008/128775
- Armstrong NA: "Tablet Manufacture by Direct Compression" In: Swarbrick J (Ed.): "Encyclopedia of Pharmaceutical Technology", 2007, Informa Healthcare USA, Inc, New York ISBN: 0-8493-9399-X vol. 6, pages 3673-3683,

## Description

### FIELD OF THE INVENTION

The present invention provides a solid pharmaceutical composition in the form of immediate release tablets, comprising pregabalin particles having specific particle size distribution with a D(50) from 100 µm to 420 µm and/or D(90) value from 300 µm to 850 µm, having good dissolution profile and good stability. The invention also encompasses a process for preparing the solid pharmaceutical composition in the form of immediate release tablets by direct compression.

### BACKGROUND OF THE INVENTION

Pregabalin, chemically described as (S)-(+)-3-aminometyl-5-methyl-1-hexanoic acid and depicted in figure 1, is a γ-aminobutyric acid or (S)-3-isobutyl (GABA) analogue. Pregabalin is commercialized as hard capsules for immediate release, under the brand name Lyrica® by Pfizer, for the treatment of Neuropathic Pain, Epilepsy and Generalised Anxiety Disorder (GAD).

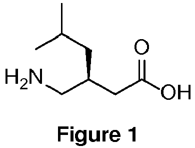

It is known that pregabalin is chemically unstable during synthesis and/or formulation and/or storage. One significant problem of pregabalin is the formation of toxic impurities such as the corresponding cyclic lactam 4-isobutyl-pyrrolidin-2-one formed from cyclisation of the γ-amino acid. In formulations, many excipients tend to react with pregabalin to form the corresponding lactam if water and/or organic solvents are present in the manufacturing formulation process. Also, common impurities and/or residues present in excipients can interact with pregabalin producing further impurities.

WO2002/078747 discloses a composition in the form of a tablet comprising previously synthesised pregabalin lactose conjugates, lactose, cornstarch and magnesium stearate prepared by wet granulation methods. According to Journal of Pharmaceutical and Biomedical Analysis, vol.28, 2002, pages 917-924, these pregabalin conjugates are, in fact, considered as degradation products. Therefore, it is desirable to provide pharmaceutical compositions having a low percentage of degradation products which do not comprise pregabalin conjugate forming excipients.

EP1077691 relates to tablets, powders and granules comprising gabapentin or/and pregabalin containing a humectant as a stabilizer. Particularly, EP1077691 also describes powders and granules compositions prepared by wet granulation comprising pregabalin, a humectant such as polyethylene glycol or butylene glycol, and hydroxypropyl cellulose as an auxiliary agent. The powders and granules containing these humectants showed a lower content of the lactam impurity when compared to powders and granules comprising pregabalin and the cellulose derivate under storage conditions at 60 °C after 4 weeks.

EP1077692 describes compositions comprising pregabalin, an α-amino acid and an auxiliary agent. The α-amino acid is used as stabilizer in the composition since prevents the cyclisation of pregabalin during the wet and/or dry granulation process and storage. Specifically, compacted powders comprising pregabalin and L-leucine (a-amino acid) are described, showing a reduction in the content of the lactam impurity when compared to samples containing magnesium stearate or talc instead of the α-amino acid. According to EP1077692, the α-amino acid is considered essential to the manufacture of pregabalin compositions having a low content of lactam impurity. It is also disclosed that the α-amino acid has a better lubricant effect than magnesium stearate.

Pregabalin has very poor compression properties, which makes the manufacture of pharmaceutical compositions via a direct compression process difficult as stated in EP1077691 and EP1077692. Thus granulation was the method of choice to provide pharmaceutical compositions comprising pregabalin and a stabilizer such as a humectant or an α-amino acid with the purpose of manufacturing pharmaceutical compositions comprising pregabalin having a low content of the lactam impurity. WO2008/128775 describes compositions comprising pregabalin essentially free from saccharides and comprising no further amino acids, apart from pregabalin. According to WO2008/128775 saccharides, such as microcrystalline cellulose, can lead to the formation of undesired pregabalin conjugates. In addition, particularly, WO2008/128775 describes tablets comprising pregabalin having a particle size of D(50) of 5 µm, calcium hydrogen phosphate (as a filler), kollidon (as a disintegrant), talc and magnesium stearate (as lubricants) prepared by direct compression (see example 3b). However, neither stability data of the composition nor any data related to the dissolution profile of these tablets are disclosed. WO2008/128775 also cites that pregabalin having different particle size distribution ranges can be used to prepare pregabalin compositions such as tablets, capsules or powders. The ranges of particle size disclosed vary from a D(50) less than 250 µm, preferably form 10 to 150 µm, another range having a D(50) from 0.01 to 50 µm, preferably from 2 to 5 µm; a further range having a D(50) above 5 µm; and a D(50) of more than 250 µm to 1.3 mm.

It is particularly desirable to provide a composition suitable for immediate release which is stable insofar as it has a very low amount of impurities and/or degradation products, particularly of the toxic lactam after storage conditions and which has a good dissolution profile and bioavailability. In addition, it is the object of the present invention to provide a composition having good chemical and mechanical properties such as good uniformity, friability, hardness (resistant to crushing), and disintegration time.

### BRIEF DESCRIPTION OF THE INVENTION

Within the context of this patent application, the expression "pregabalin" comprises the S-isomer, the R-isomer and/or a R/S-isomer mixture. In a preferred embodiment, "pregabalin" consists of (S)-pregabalin.

The present inventors have surprisingly found a solid pharmaceutical composition in the form of immediate release tablets, comprising pregabalin particles having specific particle size distribution with a D(50) from 100 µm to 420 µm and/or D(90) from 300 µm to 850 µm, having good dissolution profile and good stability.

Thus, a first aspect of the present invention provides a solid pharmaceutical composition in the form of tablets for immediate release, comprising pregabalin particles characterized by having a particle size distribution with a D(50) from 100 µm to 420 µm and/or a D(90) from 300 µm to 850 µm, both measured by volume, at least one filler and at least one lubricant, wherein the solid pharmaceutical composition is prepared by direct compression and wherein the filler is a cellulose derivative. Preferably the lubricant is not an α-amino acid.

A second aspect of the present invention provides a process for preparing the solid pharmaceutical composition according to the first aspect by direct compression.

### DEFINITIONS

The term "particle size distribution" (PSD) as used herein refers to the relative percentages by volume of each of the different size fractions of a particulate matter. The particle size distributions of the present application can be measured using laser light diffraction equipment, such as Malvern Mastersizer® 2000. Particle size is determined by measuring the angular distribution of laser light scattered by a homogeneous suspension of particles. The size distribution is determined from the light scattering data using the theory of light scattering developed by Gustav Mie. Other types of equipment are also suitable to determine particle size distribution. Laser light diffraction results can be expressed by D(90), and/or D(50) median particle size values, which are based on a volume distribution. The D(50) is the size in microns that splits the distribution with half above and half below this diameter. The term "Dx", as used herein, means that x% of the particles in a composition (based on volume) have a diameter equal to or below a specified D value. Thus, a D(90) of 300 µm means that 90% by volume, of the particles, have a diameter equal to or below 300 µm. In addition to using the D(90) value as a measuring reference to determine the particle size, D(50) is also used for such a purpose. Likewise, a D(50) of 150 µm means that 50% by volume, of the particles, have a diameter equal to or below 150 µm.

The term "dosage uniformity" as used herein refers to the degree of uniformity in the amount of the active substance among dosage units; defining dosage units as dosage forms containing a single dose or a part of a dose of the active substance in each dosage unit. The dosage uniformity of the pharmaceutical compositions according to the present invention can be demonstrated by means of performing the test for content uniformity, as states in European Pharmacopeia 8.5 (2.9.40, monograph). As defined therein, the requirements for dosage uniformity are met if the acceptance value (AV) of the dosage units is less than or equal to 15. The acceptance value (AV) is a figure which characterizes the dosage uniformity. The acceptance value is calculated, according to the European Pharmacopeia 8.5 (2.9.40, monograph).

The term "flowability" as used herein refers to the ability of divided solids (for example powders and granules) to flow vertically under defined conditions. As stated in European Pharmacopeia 8.5 (2.9.16, monograph) the flowability is determined by measuring the time needed for a test sample to flow out of a funnel, which can have different angle and/or orifice diameter according to the flow properties of the material to be tested. Typical orifice diameters of the funnel are 10 mm, 15 mm and 25 mm. The flowability is expressed in seconds and tenths of seconds, related to 100 g of sample.

The term "friability" refers to an index which provides a measure of the ability of a tablet to withstand both shock and abrasion without crumbling when handling during manufacturing, packaging, shipping and consumer use. The friability of the tablets is performed according to the equipment and method described in the European Pharmacopeia 8.5 (2.9.7, monograph).

The term "saccharide" should commonly be understood to mean sugar with a hydroxyaldehyde or hydroxyketone structure. The term "saccharide" comprises in general monosaccharides, disaccharides and polysaccharides. The term "monosaccharide" comprises the pentoses arabinose, ribose, xylose and the hexoses glucose, mannose, galactose and fructose. The term "disaccharide" comprises sucrose, trehalose, lactose and maltose. The term "polysaccharide" comprises starch, glycogen and cellulose and cellulose ethers such as e.g. ethyl cellulose, carboxymethylcellulose, hydroxypropylmethylcellulose (HPMC) and hydroxypropylcellulose.

The term "filler" as used herein is defined as an agent able to increase the bulk of a solid pharmaceutical composition, and may make a pharmaceutical dosage form containing the composition easier to be handled by a patient and/or a care-giver. The term "lubricant" as used herein is defined as an agent able to decrease adhesion of a powder to punches and friction between particles.

### DETAILED DESCRIPTION OF THE INVENTION

The present inventors have surprisingly found a solid pharmaceutical composition in the form of immediate release tablets, comprising pregabalin particles having a specific particle size distribution with a D(50) from 100 µm to 420 µm and/or a D(90) from 300 µm to 850 µm, both measured by volume, at least one filler and at least one lubricant, wherein the tablets are prepared by direct compression, and wherein the filler is a cellulose derivative.

The D(50) value is preferably in the range from 120 to 400 µm. The D(90) value is preferably in the range from 320 to 800 µm.

In one embodiment the solid pharmaceutical composition in the form of immediate release tablets, comprises pregabalin particles having a specific particle size distribution with a D(50) from 100 to 420 µm.

In another embodiment the solid pharmaceutical composition in the form of immediate release tablets, comprises pregabalin particles having a specific particle size distribution with a D(90) from 300 µm to 850 µm.

In another embodiment the solid pharmaceutical composition in the form of immediate release tablets, comprises pregabalin particles having a specific particle size distribution with a D(50) from 100 to 420 µm and a D(90) from 300 µm to 850 µm.

In one embodiment the solid pharmaceutical composition in the form of immediate release tablets, comprises pregabalin particles having a specific particle size distribution with a D(50) from 120 to 400 µm.

In another embodiment the solid pharmaceutical composition in the form of immediate release tablets, comprises pregabalin particles having a specific particle size distribution with a D(90) from 320 µm to 800 µm.

In another embodiment the solid pharmaceutical composition in the form of immediate release tablets, comprises pregabalin particles having a specific particle size distribution with a D(50) from 120 to 400 µm and a D(90) from 320 µm to 800 µm.

In another embodiment the solid pharmaceutical composition in the form of immediate release tablets, comprises pregabalin particles having a specific particle size distribution with a D(50) from 100 to 420 µm and a D(90) from 320 µm to 800 µm.

In another embodiment the solid pharmaceutical composition in the form of immediate release tablets, comprises pregabalin particles having a specific particle size distribution with a D(50) from 120 to 400 µm and a D(90) from 300 µm to 850 µm.

The solid pharmaceutical composition in the form of immediate release tablets of the present invention has a good hardness, friability, disintegration time and a good uniformity of the content as well as a good dissolution profile and a low content of impurities and related substances.

The solid pharmaceutical compositions in the form of immediate release tablets of the first aspect do not require the use of stabilizers disclosed in the art for stabilization. Reducing the number of excipients used can reduce the steps and costs of the manufacturing formulation process. In addition, solid pharmaceutical compositions in the form of immediate release tablets of the first aspect prepared by direct compression provide compositions having good dissolution profiles and a low concentration of impurities and related substances, when compared to granulation methods.

According to the first aspect of the invention, it is provided a solid pharmaceutical composition in the form of immediate release tablets, comprising:
i. pregabalin particles having a particle size distribution with a D(50) from 100 µm to 420 µm,
ii. at least one filler, and
iii. at least one lubricant;
wherein the tablets are prepared by direct compression, and wherein the filler is a cellulose derivative.

According to a preferred aspect of the invention, it is provided a solid pharmaceutical composition in the form of immediate release tablets, comprising:
i. pregabalin particles having a particle size distribution with a D(50) from 120 µm to 400 µm,
ii. at least one filler, and
iii. at least one lubricant;
wherein the tablets are prepared by direct compression, and wherein the filler is a cellulose derivative.

In an embodiment, the solid pharmaceutical composition according to the first aspect relates to immediate release tablets comprising:
i. pregabalin particles having a particle size distribution with a D(50) from 100 µm to 420 µm and/or a D(90) from 300 µm to 850 µm,
ii. at least one filler, and
iii. at least one lubricant;
wherein the tablets are prepared by direct compression, and wherein the filler is a cellulose derivative.

In an embodiment, the solid pharmaceutical composition according to the first aspect relates to immediate release tablets comprising:
i. pregabalin particles having a particle size distribution with a D(50) from 120 µm to 400 µm and/or a D(90) from 320 µm to 800 µm,
ii. at least one filler, and
iii. at least one lubricant;
wherein the tablets are prepared by direct compression, and wherein the filler is a cellulose derivative.

The fillers (ii) are cellulose derivatives, such as cellulose powder, microcrystalline cellulose, silicified microcrystalline cellulose, hydroxypropyl cellulose, hydroxypropyl methyl cellulose.

Preferably, the filler is a cellulose derivate selected from methyl cellulose, microcrystalline cellulose, silicified microcrystalline cellulose, hydroxypropyl cellulose, hydroxypropyl methyl cellulose and mixtures thereof; more preferably the filler is microcrystalline cellulose, as the impurity and related substances content is reduced and the dissolution profile and the compactability of the composition is improved. Preferably, the tablet of the first aspect does not contain lactose or an alkaline earth phosphate.

Preferably, the filler (ii) may be characterized by having a particle size distribution of D(50) below 200 µm. More preferably, the filler (ii) may be characterized by having a particle size distribution of D(50) below 150 µm. The filler (ii) of the first aspect may be present in an amount from 45% to 80% by weight relative to the total weight of the composition. Preferably, the filler may be present in an amount from 45% to 70% by weight relative to the total weight of the composition, as improves the compactability of the composition.

In an embodiment, the solid pharmaceutical composition according to the first aspect relates to immediate release tablets comprising:
i. pregabalin particles having a particle size distribution with a D(50) from 100 µm to 420 µm and/or a D(90) from 300 µm to 850 µm,
ii. at least one filler, wherein the filler is a cellulose derivative
iii. at least one lubricant;
wherein the tablets are prepared by direct compression, preferably the tablet does not contain lactose.

In an embodiment, the solid pharmaceutical composition according to the first aspect relates to immediate release tablets comprising:
i. pregabalin particles having a particle size distribution with a D(50) from 100 µm to 420 µm and/or a D(90) from 300 µm to 850 µm,
ii. at least one filler, wherein the filler is a cellulose derivative selected from methyl cellulose, microcrystalline cellulose, silicified microcrystalline cellulose, hydroxypropyl cellulose, hydroxypropyl methyl cellulose, and mixtures thereof.
iii. at least one lubricant;
wherein the tablets are prepared by direct compression, preferably the tablet does not contain lactose.

In an embodiment, the solid pharmaceutical composition according to the first aspect relates to immediate release tablets comprising:
i. pregabalin particles having a particle size distribution with a D(50) from 100 µm to 420 µm and/or a D(90) from 300 µm to 850 µm,
ii. at least one filler, wherein the filler is a cellulose derivative characterized by having a particle size distribution of D(50) below 200 µm.
iii. at least one lubricant;
wherein the tablets are prepared by direct compression, preferably the tablet does not contain lactose.

In an embodiment, the solid pharmaceutical composition according to the first aspect relates to immediate release tablets comprising:
i. pregabalin particles having a particle size distribution with a D(50) from 100 µm to 420 µm and/or a D(90) from 300 µm to 850 µm,
ii. at least one filler, wherein the filler is a cellulose derivative and is present in an amount from 45% to 80%, preferably from 45 to 70% by weight relative to the total weight of the composition.
iii. at least one lubricant;
wherein the tablets are prepared by direct compression, preferably the tablet does not contain lactose.

Suitable lubricants (iii) of the first aspect are alkaline stearate lubricants such as magnesium stearate, calcium stearate and zinc stearate, sodium stearyl fumarate, sodium lauryl sulphate, sucrose stearate, glyceryl palmitostearate, sodium benzoate, talc, and mixtures thereof. Preferably, the lubricant is alkaline stearate selected from magnesium stearate, calcium stearate and zinc stearate, preferably the lubricant is magnesium stearate. Preferably, the lubricant is not an α-amino acid. The lubricant may be present in an amount from 0.1% to 5% by weight relative to the total weight of the composition. Preferably, the lubricants are present in an amount from 0.1% to 2% to the total weight of the composition.

Suitable pregabalin particles (i) of the first aspect are in crystalline solid form. Preferably, the crystalline solid form of pregabalin particles (i) corresponds to crystalline solid form I as disclosed in US2006/270871, characterized by having an X-Ray Powder Diffraction (PXRD) pattern with the following characteristic degrees two theta values at 9.5, 16.62, 18.18, 18.32, 19.06, 19.74, 22.14 and 35.62 ± 0.2.

Advantageously, tablets prepared by direct compression having a high load of the active ingredient, pregabalin, were prepared, allowing the manufacturing of tablets which are easier to swallow. Suitable pregabalin particles (i) of the first aspect may be present in an amount from 15% to 50% by weight relative to the total weight of the tablets. Preferably, pregabalin particles (i) of the first aspect may be present in an amount from 25% to 45% by weight relative to the total weight of the tablets.

In an embodiment, the solid pharmaceutical composition according to the first aspect relates to immediate release tablets comprising:
i. pregabalin particles having a particle size distribution with a D(50) from 100 µm to 420 µm and/or a D(90) from 300 µm to 850 µm,
ii. at least one filler, wherein the filler is a cellulose derivative and is present in an amount from 45% to 80%, preferably from 45 to 70 % by weight relative to the total weight of the composition.
iii. at least one lubricant; wherein the lubricant is an alkaline stearate selected from magnesium stearate, calcium stearate and zinc stearate, more preferably magnesium stearate;
wherein the tablets are prepared by direct compression. Preferably, the tablet does not contain lactose and does not contain α-amino acid.

In a particular embodiment, the solid pharmaceutical composition according to the first aspect relates to immediate release tablets comprising:
i. from 15% to 50% by weight of pregabalin particles characterized by having a particle size distribution with a D(50) from 100 µm to 420 µm and/or a D(90) from 300 µm to 850 µm,
ii. from 45% to 80% by weight of at least one filler, and
iii. from 0.1% to 5% by weight of at least one lubricant,
wherein the percentage by weight is relative to the total weight of the composition and wherein the immediate release tablets are prepared by direct compression, and wherein the filler is a cellulose derivative.

Preferably, the tablet does not contain lactose and does not contain α-amino acid.

In a particular embodiment, the solid pharmaceutical composition according to the first aspect relates to immediate release tablets comprising:
i. from 25% to 45% by weight of pregabalin particles characterized by having a particle size distribution with a D(50) from 100 µm to 420 µm and/or a D(90) from 300 µm to 850 µm,
ii. from 45% to 70 % by weight of at least one filler, and
iii. from 0.1% to 5% by weight of at least one lubricant,
wherein the percentage by weight is relative to the total weight of the composition and wherein the immediate release tablets are prepared by direct compression, and wherein the filler is a cellulose derivative.

Preferably, the tablet does not contain lactose and does not contain α-amino acid.

In a more particular embodiment, the solid pharmaceutical composition according to the first aspect relates to immediate release tablets, comprising:
i. from 25% to 45% by weight of pregabalin particles characterized by having a particle size distribution with a D(50) from 100 µm to 420 µm and/or D(90) from 300 µm to 850 µm,
ii. from 45% to 70% by weight of at least one filler, and
iii. from 0.1% to 2% by weight of at least one lubricant,
wherein the percentage by weight is relative to the total weight of the composition and wherein the immediate release tablets are prepared by direct compression and wherein the filler is a cellulose derivative and wherein the lubricant is not an α-amino acid.

Preferably, the tablet does not contain lactose and does not contain α-amino acid.

The inventors have surprisingly found that solid pharmaceutical compositions in the form of immediate release tablets prepared by direct compression comprising pregabalin particles having a particle size distribution with a D(50) lower than 100 µm and/or a D(90) lower than 300 µm provided tablets with sticking problems, leading to the composition being adhered to the punches during the compression process and resulting in tablets having very poor uniformity. In addition, pregabalin particles having a particle size distribution with a D(50) lower than 100 µm and/or a D(90) lower than 300 µm also showed sticking problems, poor uniformity and very low flowability. These results are surprising because when using direct compression processes usually the active ingredients employed have a particle size distribution with a D(90) below 70 µm and/or a D(50) below 35 in order to prevent and avoid segregation and uniformity content problems when mixed with excipients. Surprisingly, the inventors have also found that solid pharmaceutical compositions in the form of immediate release tablets prepared by direct compression comprising pregabalin particles having a particle size distribution with a D(50) from 100 to 420 µm and/or a D(90) from 300 to 850 µm, provide tablets with good uniformity for all dosage forms, good friability, and good hardness. In addition, these solid pharmaceutical compositions show good dissolution profiles and a low concentration of impurities and related substances.

Moreover, the compositions prepared by direct compression in the form of immediate release tablets comprising pregabalin particles having a particle size distribution with a D(50) higher than 420 and/or a D(90) higher than 850 µm provided tablets with uniformity content problems.

The solid pharmaceutical compositions in the form of immediate release tablets according to the first aspect are suitable to prepare various dosage forms, including 25 mg, 50 mg, 75 mg, 100 mg, 150 mg, 200 mg, 225 mg and 300 mg, providing good uniformity content for all dosages.

The solid pharmaceutical compositions in the form of immediate release tablets according to the first aspect show an improved friability, with less than 1%. Preferably, the solid pharmaceutical composition of the first aspect has a friability of less than 0.3%. More preferably, the solid pharmaceutical composition of the first aspect has a friability of less than 0.1%.

The solid pharmaceutical compositions in the form of immediate release tablets of the first aspect show an improved dissolution profile and also excellent storage stability having a low amount of impurities and related substances over a storage period of 3 months. The solid pharmaceutical compositions in the form of immediate release tablets of the first aspect further show an improved dissolution profile and also excellent long term storage stability having a low amount of impurities and related substances over a storage period of 3 - 12 months (see Examples 6 - 10). Preferably, the solid pharmaceutical composition of the present invention contains less than 0.15% by weight of the lactam impurity, more preferably less than 0.10% by weight of the lactam impurity, more preferably less than 0.05% of the lactam impurity. Also, the solid pharmaceutical compositions of the first aspect contain less than 0.20% by weight of total degradation products, based on the total weight of the composition, preferably less than 0.15% by weight of the of total degradation products, based on the total weight of the composition, more preferably less than 0.10% by weight of total degradation products, based on the total weight of the composition.

The tablets according to the first aspect can be coated with a functional polymer which acts as a filmogenic agent. The functional polymer can be any polymer suitable for film coating.

The second aspect of the present invention relates to a process for preparing the solid pharmaceutical composition in the form of immediate release tablets according to the first aspect by direct compression, comprising at least the following steps:
a) Providing pregabalin particles having a particle size distribution with a D(50) from 100 to 420 µm and/or a D(90) from 300 µm to 850 µm and at least one filler, wherein the filler is a cellulose derivative;
b) Mixing the ingredients of step a) in a suitable mixer to achieve a homogeneous mixture;
c) Mixing the blend of step b) with at least one lubricant, until a homogeneous powder is obtained;
d) Compressing the powder mixture of step c) into tablets;
e) Optionally, coating the tablets of step d), with a functional polymer which acts as a filmogenic agent.

A functional polymer can be any polymer suitable for film coating.

Preferably the pregabalin particles of step a) have a D(50) from 120 to 400 µm and/or a D(90) from 320 µm to 800 µm.

All ingredients such as pregabalin, the filler and the lubricant being compressed according to the second aspect may be suitable sifted through appropriate sieves to reduce or avoid the formation of agglomerates before the blending steps a) and/or b). Ingredients may be sieved using a suitable mesh, having a mesh size of from 2.5 mm to 0.20 mm.

Advantageously, the solid pharmaceutical composition in the form of immediate release tablets obtained according to the second aspect show a very low content of total impurities and related substances as well as low content of the toxic lactam impurity.

Advantageously, the solid pharmaceutical composition in the form of immediate release tablets obtained according to the second aspect shows a very low content of total degradation products of preferably less than 0.10% by weight, based on the total content of the composition and a low content of the toxic lactam impurity of preferably less than 0.10% by weight, based on the total content of the composition both after 3 and 6 months storage at a temperature of 40°C and 75% relative humidity (RH) (see Examples 6, 7, and 9). Further, the solid pharmaceutical composition in the form of immediate release tablets obtained according to the second aspect shows a very low content of total degradation products of preferably less than 0.10% by weight, based on the total content of the composition and a low content of the toxic lactam impurity of preferably less than 0.10% by weight, based on the total content of the composition after 3, 6, 9 and 12 months storage at a temperature of 25°C and 60% relative humidity (RH) (see Examples 8 and 10).

The inventors have found a higher content of impurities were generated when the solid pharmaceutical compositions were prepared by granulation process, particularly wet granulation when compared to the direct compression process.

### EXPERIMENTAL

### General methods

### Particle Size Distribution (PSD)

The determination of the particle size by Laser light diffraction is carried out using a Malvern Mastersizer 2000 particle analyzer.

### Method parameters:

Sample handling unit: Hydro 2000SM(A)
Particle RI: 1.520
Dispersant name: Heptane + 0.2% Span 20
Dispersant RI: 1.390
Particle RI: 1.520
Analysis model: General purpose
Result range: 0.02 µm to 2000 µm
Obscuration limits: 10 - 20 %
Reagents: Heptane. Dilution of 0.2 % of Span 20 in heptane

### Samples preparation

Transfer about 100 mg of sample to a small mortar, add about 1 mL of heptane + 0.2% Span 20 and dampen it with the aid of a spatula.

### Execution

Rinse the accessory Hydro 2000SM with heptane for at least 2 times. Perform an automatic alignment and once the alignment displayed is stable, obtain a blank lecture with heptane and correct. Place a suitable amount of sample, with the aid of a pipette, to obtain a good obscuration (between 10 - 20%). As soon as the desired obscuration is reached, perform the sample measurement, analyze the data and display the result.

### Examples

### Example 1. Solid pharmaceutical composition in the form of immediate release tablets containing (S)-pregabalin having a D(50): 137 µm and a D(90): 351 µm

| **Component** | **Function** | **Amount (mg per tablet)** | | | | **Percentage (% w/w)** |
|---|---|---|---|---|---|---|
| (S)-Pregabalin | Active ingredient | 25.00 | 75.00 | 150.00 | 300.00 | 39.68 |
| Cellulose microcrystalline | Filler | 37.37 | 112.11 | 224.22 | 448.44 | 59.32 |
| Magnesium stearate | Lubricant | 0.63 | 1.89 | 3.78 | 7.56 | 1.00 |
| Total weight per tablet | | 63.00 | 189.00 | 378.00 | 756.00 | 100.00 |

### Example 2. Solid pharmaceutical composition in the form of immediate release tablets containing (S)-pregabalin having a D(50): 261 µm and a D(90): 471 µm.

| **Component** | **Function** | **Amount (mg per tablet)** | | | | **Percentage (% w/w)** |
|---|---|---|---|---|---|---|
| (S)-Pregabalin | Active ingredient | 25.00 | 75.00 | 150.00 | 300.00 | 39.68 |
| Cellulose microcrystalline | Filler | 37.37 | 112.11 | 224.22 | 448.44 | 59.32 |
| Magnesium stearate | Lubricant | 0.63 | 1.89 | 3.78 | 7.56 | 1.00 |
| Total weight per tablet | | 63.00 | 189.00 | 378.00 | 756.00 | 100.00 |

The general manufacturing process via direct compression is as follows:
a) The (S)-pregabalin particles and microcrystalline cellulose are weighed and sieved through a suitable mesh.
b) Both, pregabalin and microcrystalline cellulose are mixed in a suitable mixer to achieve a homogeneous mixture.
c) After weighing and sieving the magnesium stearate through a suitable mesh it is incorporated into the homogeneous mixture obtained in step b). Said blend is mixed until a homogenous mixture is obtained.
d) Compress the mixture of powder obtain in step c) in a tableting machine equipped with the suitable punches.

### Example 3. Immediate release tablet containing 300 mg of (S)-pregabalin having a PSD with a D(90) of 351 µm

(S)-Pregabalin particles (300 mg dose) having a PSD with a D(90) of 351 µm and D(50) 137 µm were blended with cellulose microcrystalline Avicel® PH-102 (448.44 mg) for 30 minutes in a diffusion mixer. Magnesium stearate (7.56 mg) was then added to the mixture which was further mixed for 5 minutes. The obtained blend was compressed in a rotary tableting machine with 20 mm x 8 mm punches. The results of the immediate release tablet prepared are shown below.

| **PARAMETER** | **RESULT** |
|---|---|
| Disintegration time (min) | 0.3 |
| Friability (%) | < 0.1 |
| Hardness (N) | 123 |
| Assay (%) | 100.1 |
| Uniformity of dosage units | AV=3.3 |
| Lactam compound (%) | < 0.1 |
| Any unspecified degradation product (%) | ≤ 0.1 |
| Total degradation products (%) | < 0.1 |

In the present examples, the percentage of friability is the percentage of weight loss after the friability test according to the equipment and method described in the Ph. Eur. 2.9.7. The percentage of assay is the percentage of pregabalin by weight present in the tablet dosage form determined by HPLC. All other percentages are by weight.

### Example 4. Preparation according to example 1. Immediate release tablet with (S)-pregabalin having a PSD with a D(90) of 794 µm.

(S)-Pregabalin particles having a PSD with a D(90) of 794 µm (300 mg) and D(50) 382 µm were blended with cellulose microcrystalline Avicel® PH-102 (448.44 mg) for 30 minutes in a diffusion mixer. Magnesium stearate (7.56 mg) was then added to the mixture which was further mixed for 5 minutes. The obtained blend was compressed in a rotary tableting machine with 20 mm x 8 mm punches. The results of the immediate release tablet prepared are shown below.

| **PARAMETER** | **RESULT** |
|---|---|
| Disintegration time (min) | 0.2 |
| Friability (%) | 0.1 |
| Hardness (N) | 149 |
| Assay (%) | 99.7 |
| Uniformity of dosage units | AV=1.2 |
| Lactam compound (%) | < 0.1 |
| Any unspecified degradation product (%) | ≤ 0.1 |
| Total degradation products (%) | < 0.1 |

### Example 5. Dissolution profile

Dissolution profile data for (S)-pregabalin tablets of the present invention and the commercial Lyrica® capsules. Conditions: 900 mL HCl 0.1 M, 50 rpm paddle.

| | **LYRICA® capsules** | | **Tablet composition of example 3** | | **Tablet composition of example 4** | |
|---|---|---|---|---|---|---|
| **Dose (mg)** | 300 | | 300 | | 300 | |
| **Time (s)** | **% Drug release** | **RSD** | **% Drug release** | **RSD** | **% Drug release** | **RSD** |
| 0 | 0 | 0 | 0 | 0 | 0 | 0 |
| 10 | 89 | 7.3 | 97 | 3.2 | 102 | 1.7 |
| 15 | 94 | 5.5 | 99 | 2.8 | 103 | 1.6 |
| 20 | 96 | 4.5 | 100 | 2.5 | 104 | 1.2 |
| 30 | 99 | 2.7 | 101 | 2.2 | 104 | 1.3 |

| | | | | | | |
|---|---|---|---|---|---|---|
| RSD: Relative Standard Deviation | | | | | | |

### Example 6. Stability experiments

Stability data of pregabalin tablets of 300 mg prepared as example 2, packed in an Aluminium-Aluminium blister, upon stability conditions at 40°C/75%RH after a period of 1 month, 2 months and 3 months, is shown below.

| **PARAMETER** | **RESULTS** | | |
|---|---|---|---|
| | **1 month** | **2 months** | **3 months** |
| Disintegration time (min) | 0.2 | 0.2 | 0.2 |

| | | | |
|---|---|---|---|
| Hardness (N) | 118 | 120 | 117 |
| Assay (%) | 99.1 | 100.8 | 99.1 |
| Lactam compound (%) | < 0.1 | < 0.1 | 0.1 |
| Any unspecified degradation product (%) | ≤ 0.1 | ≤ 0.1 | ≤ 0.1 |
| Total degradation products (%) | < 0.1 | < 0.1 | 0.1 |

### Example 7. Stability experiments

Stability data of pregabalin tablets of 300 mg prepared as example 4, packed in an Aluminium-Aluminium blister, upon stability conditions at 40°C/75%RH is shown below.

| **PARAMETER** | **RESULTS** | | |
|---|---|---|---|
| | **0 month** | **3 months** | **6 months** |
| Disintegration time (min) | 0.2 | 0.2 | 0.2 |
| Hardness (N) | 149 | 136 | 135 |
| Assay (%) | 99.7 | 101.2 | 101.6 |
| Lactam compound (%) | < 0.1 | 0.1 | 0.1 |
| Any unspecified degradation product (%) | ≤ 0.1 | ≤ 0.1 | ≤ 0.1 |
| Total degradation products (%) | < 0.1 | 0.1 | 0.1 |

### Example 8. Stability experiments

Stability data of pregabalin tablets of 300 mg prepared as example 4, packed in an Aluminium-Aluminium blister, upon stability conditions at 25°C/60%RH is shown below.

| **PARAMETER** | RESULTS | | | | |
|---|---|---|---|---|---|
| | 0 month | 3 months | 6 months | 9 months | 12 months |
| Disintegration time (min) | 0.2 | 0.2 | 0.2 | 0.3 | 0.2 |
| Hardness (N) | 149 | 149 | 146 | 143 | 141 |
| Assay (%) | 99.7 | 101.3 | 102.1 | 101.3 | 102.2 |
| Lactam compound (%) | < 0.1 | < 0.1 | 0.1 | 0.1 | 0.1 |
| Any unspecified degradation product (%) | ≤ 0.1 | ≤ 0.1 | ≤ 0.1 | ≤ 0.1 | ≤ 0.1 |
| Total degradation products (%) | < 0.1 | < 0.1 | 0.1 | 0.1 | 0.1 |

### Example 9. Stability experiments

Stability data of pregabalin tablets of 25 mg prepared as example 2, packed in an Aluminium-Aluminium blister, upon stability conditions at 40°C/75%RH is shown below.

| **PARAMETER** | **RESULTS** | | |
|---|---|---|---|
| | **0 month** | **3 months** | **6 months** |
| Disintegration time (min) | 0.1 | 0.1 | 0.1 |
| Hardness (N) | 45 | 42 | 43 |
| Assay (%) | 98.0 | 99.8 | 100.7 |
| Lactam compound (%) | < 0.1 | 0.1 | 0.1 |
| Any unspecified degradation product (%) | ≤ 0.1 | ≤ 0.1 | ≤ 0.1 |
| Total degradation products (%) | < 0.1 | 0.1 | 0.1 |

### Example 10. Stability experiments

Stability data of pregabalin tablets of 25 mg prepared as example 2, packed in an Aluminium-Aluminium blister, upon stability conditions at 25°C/60%RH is shown below.

| **PARAMETER** | **RESULTS** | | | | |
|---|---|---|---|---|---|
| | **0 month** | **3 months** | **6 months** | **9 months** | **12 months** |
| Disintegration time (min) | 0.1 | 0.2 | 0.1 | 0.2 | 0.2 |
| Hardness (N) | 45 | 44 | 43 | 42 | 39 |
| Assay (%) | 98.0 | 100.3 | 99.1 | 101.3 | 99.8 |
| Lactam compound (%) | < 0.1 | < 0.1 | 0.1 | 0.1 | 0.1 |
| Any unspecified degradation product (%) | ≤ 0.1 | ≤ 0.1 | ≤ 0.1 | ≤ 0.1 | ≤ 0.1 |
| Total degradation products (%) | < 0.1 | < 0.1 | 0.1 | 0.1 | 0.1 |

## Claims

1. A solid pharmaceutical composition in the form of an immediate release tablet, comprising:
i. pregabalin particles having a particle size distribution with a D(50) from 100 µm to 420 µm, and/or pregabalin particles having a particle size distribution with a D(90) from 300 µm to 850 µm, both measured by volume,
ii. at least one filler, and
iii. at least one lubricant,
wherein the immediate release tablet is prepared by direct compression, and
wherein the filler (ii) is a cellulose derivative.

2. The solid pharmaceutical composition according to claim 1, wherein the pregabalin particles (i) are **characterized by** having a particle size distribution with a D(50) from 120 µm to 400 µm.

3. The solid pharmaceutical composition according to any of the preceding claims, wherein the pregabalin particles (i) are **characterised by** having a particle size distribution with D(90) from 320 µm to 800 µm.

4. The solid pharmaceutical composition according to any of the preceding claims, wherein the cellulose derivative is **characterized by** having particles size distribution of D(50) below 200 µm.

5. The solid pharmaceutical composition according to claim 4, wherein the cellulose derivative is selected from methyl cellulose, microcrystalline cellulose, hydroxypropyl cellulose, hydroxypropyl methyl cellulose, and mixtures thereof, preferably the cellulose derivate is microcrystalline cellulose.

6. The solid pharmaceutical composition according to any the preceding claims, wherein the lubricant (iii) is an alkaline stearate.

7. The solid pharmaceutical composition according to claim 6, wherein the alkaline stearate is selected from magnesium stearate, calcium stearate and mixtures thereof, preferably the alkaline stearate is magnesium stearate.

8. The solid pharmaceutical composition according to anyone of the preceding claims, wherein the filler (ii) is present in an amount from 45% to 80% by weight, based on the total weight of the composition, preferably in an amount from 45% to 70%, based on the total weight of the composition.

9. The solid pharmaceutical composition according to any of the preceding claims, wherein the pregabalin particles (i) are present in amount from 15% to 50% by weight, based on the total weight of the composition, preferably from 25% to 45% by weight, based on the total weight of the composition.

10. The solid pharmaceutical composition according to any of the preceding claims, comprising:
i. from 15% to 50% by weight of pregabalin particles having a particle size distribution with a D(50) from 100 µm to 420 µm, and/or pregabalin particles having a particle size distribution with a D(90) from 300 µm to 850 µm, both measured by volume,
ii. from 45% to 80% by weight of at least one filler and
iii. from 0.1% to 5% by weight of at least one lubricant,
wherein the percentage by weight is relative to the total weight of the composition and wherein the immediate release tablet is prepared by direct compression, and wherein the filler (ii) is a cellulose derivative.

11. The solid pharmaceutical composition according to any of the preceding claims, comprising:
i. from 25% to 45% by weight of pregabalin particles having a particle size distribution with a D(50) from 100 µm to 420 µm, and/or pregabalin particles having a particle size distribution with a D(90) from 300 µm to 850 µm, both measured by volume,
ii. from 45% to 70% by weight of at least one filler and
iii. from 0.1% to 5% by weight of at least one lubricant,
wherein the percentage by weight is relative to the total weight of the composition and wherein the immediate release tablet is prepared by direct compression, and wherein the filler (ii) is a cellulose derivative.

12. The solid pharmaceutical composition according to any of the preceding claims, wherein the tablets are coated with a functional polymer, which acts as a filmogenic agent.

13. The solid pharmaceutical composition according to any of the preceding claims, wherein the content of degradation products is less than 0.20% by weight, preferably less than 0.10% by weight based on the total weight of the composition.

14. A process for preparing the solid pharmaceutical composition according to any of the preceding claims, comprising at least the following steps:
a) Providing pregabalin particles having a particle size distribution with a D(50) from 100 to 420 µm and/or a D(90) from 300 µm to 850 µm, both measured by volume, and at least one filler, wherein the filler (ii) is a cellulose derivative;
b) Mixing the ingredients of step a) in a suitable mixer to achieve a homogeneous mixture; and
c) Mixing the blend of step b) with at least one lubricant, until a homogeneous powder is obtained;
d) Compressing the powder mixture of step c) into tablets
e) Optionally, coating the tablets of step d), with a functional polymer which acts as a filmogenic agent.

15. A process for preparing according to claim 14, wherein the pregabalin particles of step a) have a D(50) from 120 to 400 µm and/or a D(90) from 320 µm to 800 µm.

## Patentansprüche

1. Feste pharmazeutische Zusammensetzung in Form einer Tablette mit sofortiger Freisetzung, umfassend:
i. Pregabalinpartikel mit einer Teilchengrößenverteilung mit einem D₅₀ von 100 µm bis 420 µm, und/oder Pregabalinpartikel mit einer Teilchengrößenverteilung mit einem D₉₀ von 300 µm bis 850 µm, beide gemessen nach Volumen,
ii. mindestens einen Füllstoff und
iii. mindestens ein Schmiermittel, wobei die Tablette mit sofortiger Freisetzung durch direktes Zusammenpressen hergestellt wird, und
wobei der Füllstoff (ii) ein Cellulosederivat ist.

2. Feste pharmazeutische Zusammensetzung nach Anspruch 1, wobei die Pregabalinpartikel (i) **dadurch gekennzeichnet sind, dass** sie eine Teilchengrößenverteilung mit einem D₅₀ von 120 µm bis 400 µm aufweisen.

3. Feste pharmazeutische Zusammensetzung nach einem der vorangehenden Ansprüche, wobei die Pregabalinpartikel (i) **dadurch gekennzeichnet sind, dass** sie eine Teilchengrößenverteilung mit einem D₉₀ von 320 µm bis 800 µm aufweisen.

4. Feste pharmazeutische Zusammensetzung nach einem der vorangehenden Ansprüche, wobei das Cellulosederivat **dadurch gekennzeichnet ist, dass** es eine Teilchengrößenverteilung von D₅₀ unter 200 µm aufweist.

5. Feste pharmazeutische Zusammensetzung nach Anspruch 4, wobei das Cellulosederivat ausgewählt ist aus Methylcellulose, mikrokristalliner Cellulose, Hydroxypropylcellulose, Hydroxypropylmethylcellulose und Gemischen davon, vorzugsweise ist das Cellulosederivat mikrokristalline Cellulose.

6. Feste pharmazeutische Zusammensetzung nach einem der vorangehenden Ansprüche, wobei das Schmiermittel (iii) ein Alkalistearat ist.

7. Feste pharmazeutische Zusammensetzung nach Anspruch 6, wobei das Alkalistearat ausgewählt ist aus Magnesiumstearat, Calciumstearat und Gemischen davon, vorzugsweise ist das Alkalistearat Magnesiumstearat.

8. Feste pharmazeutische Zusammensetzung nach einem der vorangehenden Ansprüche, wobei der Füllstoff (ii) in einer Menge von 45 Gew.-% bis 80 Gew.-%, bezogen auf das Gesamtgewicht der Zusammensetzung, vorzugsweise in einer Menge von 45 % bis 70 %, bezogen auf das Gesamtgewicht der Zusammensetzung, vorliegt.

9. Feste pharmazeutische Zusammensetzung nach einem der vorangehenden Ansprüche, wobei die Pregabalinpartikel (i) in einer Menge von 15 Gew.-% bis 50 Gew.-%, bezogen auf das Gesamtgewicht der Zusammensetzung, vorzugsweise von 25 Gew.-% bis 45 Gew.-%, bezogen auf das Gesamtgewicht der Zusammensetzung, vorliegen.

10. Feste pharmazeutische Zusammensetzung nach einem der vorangehenden Ansprüche, umfassend:
i. von 15 Gew.-% bis 50 Gew.-% Pregabalinpartikel mit einer Teilchengrößenverteilung mit einem D₅₀ von 100 µm bis 420 µm und/oder Pregabalinpartikel mit einer Teilchengrößenverteilung mit einem D₉₀ von 300 µm bis 850 µm, beide gemessen nach Volumen,
ii. von 45 Gew.-% bis 80 Gew.-% von mindestens einem Füllstoff und
iii. von 0,1 Gew.-% bis 5 Gew.-% von mindestens einem Schmiermittel,
wobei der prozentuale Gewichtsanteil bezüglich des Gesamtgewichts der Zusammensetzung ist und wobei die Tablette mit sofortiger Freisetzung durch direktes Zusammenpressen hergestellt wird und wobei der Füllstoff (ii) ein Cellulosederivat ist.

11. Feste pharmazeutische Zusammensetzung nach einem der vorangehenden Ansprüche, umfassend:
i. von 25 Gew.-% bis 45 Gew.-% Pregabalinpartikel mit einer Teilchengrößenverteilung mit einem D₅₀ von 100 µm bis 420 µm und/oder Pregabalinpartikel mit einer Teilchengrößenverteilung mit einem D₉₀ von 300 µm bis 850 µm, beide gemessen nach Volumen,
ii. von 45 Gew.-% bis 70 Gew.-% von mindestens einem Füllstoff und
iii. von 0,1 Gew.-% bis 5 Gew.-% von mindestens einem Schmiermittel,
wobei der prozentuale Gewichtsanteil bezüglich des Gesamtgewichts der Zusammensetzung ist und wobei die Tablette mit sofortiger Freisetzung durch direktes Zusammenpressen hergestellt wird und wobei der Füllstoff (ii) ein Cellulosederivat ist.

12. Feste pharmazeutische Zusammensetzung nach einem der vorangehenden Ansprüche, wobei die Tabletten mit einem funktionellen Polymer, das als Filmbildner dient, beschichtet sind.

13. Feste pharmazeutische Zusammensetzung nach einem der vorangehenden Ansprüche, wobei der Gehalt an Abbauprodukten weniger als 0,20 Gew.-%, vorzugsweise weniger als 0,10 Gew.-%, bezogen auf das Gesamtgewicht der Zusammensetzung, beträgt.

14. Verfahren zur Herstellung der festen pharmazeutischen Zusammensetzung nach einem der vorangehenden Ansprüche, umfassend zumindest die folgenden Schritte:
a) Bereitstellen von Pregabalinpartikeln mit einer Teilchengrößenverteilung mit einem D₅₀ von 100 µm bis 420 µm und/oder einem D₉₀ von 300 µm bis 850 µm, beide gemessen nach Volumen, und mindestens einem Füllstoff, wobei der Füllstoff (ii) ein Cellulosederivat ist;
b) Mischen der Bestandteile von Schritt a) in einem geeigneten Mischer, um ein homogenes Gemisch zu erzielen; und
c) Mischen des Gemischs von Schritt b) mit mindestens einem Schmiermittel, bis ein homogenes Pulver erhalten wird;
d) Pressen des Pulvergemischs von Schritt c) zu Tabletten;
e) gegebenenfalls, Beschichten der Tabletten von Schritt d) mit einem funktionellen Polymer, das als Filmbildner dient.

15. Verfahren zur Herstellung nach Anspruch 14, wobei die Pregabalinpartikel von Schritt a) einen D₅₀ von 120 µm bis 420 µm und/oder einen D₉₀ von 320 µm bis 800 µm aufweisen.

## Revendications

1. Composition pharmaceutique solide sous la forme d'un comprimé à libération immédiate, comprenant :
i. des particules de prégabaline ayant une distribution de taille de particule avec une D₅₀ de 100 µm à 420 µm, et/ou des particules de prégabaline ayant une distribution de taille de particule avec une D₉₀ de 300 µm à 850 µm, toutes deux mesurées en volume,
ii. au moins une charge, et
iii. au moins un lubrifiant,
le comprimé à libération immédiate étant préparé par compression directe, et la charge (ii) étant un dérivé de cellulose.

2. Composition pharmaceutique solide selon la revendication 1, dans laquelle les particules de prégabaline (i) sont caractérisées comme ayant une distribution de taille de particule avec une D₅₀ de 120 µm à 400 µm.

3. Composition pharmaceutique solide selon la revendication 1, dans laquelle les particules de prégabaline (i) sont caractérisées comme ayant une distribution de taille de particule avec une D₉₀ de 320 µm à 800 µm.

4. Composition pharmaceutique solide selon l'une quelconque des revendications précédentes, dans laquelle le dérivé de cellulose est caractérisé comme ayant une distribution de taille de particule de D₅₀ au-dessous de 200 µm.

5. Composition pharmaceutique solide selon la revendication 4, dans laquelle le dérivé de cellulose est choisi parmi la méthyl cellulose, la cellulose microcristalline, l'hydroxypropyl cellulose, l'hydroxypropyl méthyl cellulose et leurs mélanges, de préférence le dérivé de cellulose est la cellulose microcristalline.

6. Composition pharmaceutique solide selon l'une quelconque des revendications précédentes, dans laquelle le lubrifiant (iii) est un stéarate alcalin.

7. Composition pharmaceutique solide selon la revendication 6, dans laquelle le stéarate alcalin est choisi parmi le stéarate de magnésium, le stéarate de calcium et leurs mélanges, de préférence le stéarate alcalin est le stéarate de magnésium.

8. Composition pharmaceutique solide selon l'une quelconque des revendications précédentes, dans laquelle la charge (ii) est présente en une quantité de 45 % à 80 % en poids, sur la base du poids total de la composition, de préférence en une quantité de 45 % à 70 %, sur la base du poids total de la composition.

9. Composition pharmaceutique solide selon l'une quelconque des revendications précédentes, dans laquelle les particules de prégabaline (i) sont présentes en une quantité de 15 % à 50 % en poids, sur la base du poids total de la composition, de préférence de 25 % à 45 % en poids, sur la base du poids total de la composition.

10. Composition pharmaceutique solide selon l'une quelconque des revendications précédentes, comprenant :
i. de 15 % à 50 % en poids de particules de prégabaline ayant une distribution de taille de particule avec une D₅₀ de 100 µm à 420 µm, et/ou des particules de prégabaline ayant une distribution de taille de particule avec une D₉₀ de 300 µm à 850 µm, toutes deux mesurées en volume,
ii. de 45 % à 80 % en poids d'au moins une charge et
iii. de 0,1 % à 5 % en poids d'au moins un lubrifiant,
le pourcentage en poids étant par rapport au poids total de la composition et le comprimé à libération immédiate étant préparé par compression directe, et la charge (ii) étant un dérivé de cellulose.

11. Composition pharmaceutique solide selon l'une quelconque des revendications précédentes, comprenant :
i. de 25 % à 45 % en poids de particules de prégabaline ayant une distribution de taille de particule avec une D₅₀ de 100 µm à 420 µm, et/ou des particules de prégabaline ayant une distribution de taille de particule avec une D₉₀ de 300 µm à 850 µm, toutes deux mesurées en volume,
ii. de 45 % à 70 % en poids d'au moins une charge et
iii. de 0,1 % à 5 % en poids d'au moins un lubrifiant,
le pourcentage en poids étant par rapport au poids total de la composition et le comprimé à libération immédiate étant préparé par compression directe, et la charge (ii) étant un dérivé de cellulose.

12. Composition pharmaceutique solide selon l'une quelconque des revendications précédentes, dans laquelle les comprimés sont revêtus avec un polymère fonctionnel, qui agit comme un agent filmogène.

13. Composition pharmaceutique solide selon l'une quelconque des revendications précédentes, dans laquelle la teneur de produits de dégradation est de moins de 0,20 % en poids, de préférence de moins de 0,10 % en poids sur la base du poids total de la composition.

14. Procédé de préparation de la composition pharmaceutique solide selon l'une quelconque des revendications précédentes, comprenant au moins les étapes suivantes consistant à :
a) fournir des particules de prégabaline ayant une distribution de taille de particule avec une D₅₀ de 100 à 420 µm et/ou une D₉₀ de 300 µm à 850 µm, toute deux mesurées en volume, et au moins une charge, la charge (ii) étant un dérivé de cellulose ;
b) mélanger les ingrédients de l'étape a) dans un mixeur approprié pour obtenir un mélange homogène ; et
c) mélanger le mélange de l'étape b) avec au moins un lubrifiant, jusqu'à ce qu'une poudre homogène soit obtenue ;
d) comprimer le mélange de poudre de l'étape c) en comprimés ;
e) éventuellement, revêtir les comprimés de l'étape d), avec un polymère fonctionnel qui agit comme un agent filmogène.

15. Procédé de préparation selon la revendication 14, dans lequel les particules de prégabaline de l'étape a) ont une D₅₀ de 120 à 400 µm et/ou une D₉₀ de 320 à 800 µm.
